# EUROPEAN PATENT SPECIFICATION

(11) **EP 1 491 220 B1**
(45) Date of publication and mention of the grant of the patent: **15.04.2009**
(21) Application number: 03769988.1
(22) Date of filing: 29.10.2003
(51) Int. Cl.: A61L 27/46, A61L 27/38

(54) **TRANSPLANTATION MATERIAL FOR REGENERATING BIOLOGICAL TISSUE AND PROCESS FOR PRODUCING THE SAME**
TRANSPLANTATIONSMATERIAL ZUR REGENERATION VON BIOLOGISCHEM GEWEBE UND VERFAHREN ZU SEINER HERSTELLUNG
MATERIAU DE TRANSPLANTATION DESTINE A REGENERER DES TISSUS BIOLOGIQUES ET PROCEDE DE PRODUCTION CORRESPONDANT

(30) Priority: 31.10.2002 JP 2002317513
(43) Date of publication of application: 29.12.2004
(73) Proprietor: Tabata, Yasuhiko, Uji-shi, Kyoto 611-0024 (JP); Kubo, Akiko, Osaka-shi, Osaka 538-0054 (JP); Olympus Corporation, Shibuya-ku, Tokyo (JP)
(72) Inventor: TABATA, Yasuhiko, Uji-shi, Kyoto 611-0024 (JP); KUBO, Yoshinobu, deceased (JP); HOKUGO, Akishige, Tondabayashi-shi, Osaka 584-0001 (JP); KUBO, Akiko, Osaka 538-0054 (JP)
(74) Representative: von Hellfeld, Axel
(86) International application number: PCT/JP2003/013881
(87) International publication number: WO 2004/039423

(56) References cited:
- WO-A2-99/59500
- JP-A- 1 181 872
- JP-A- 4 242 658
- JP-A- 7 124 241
- JP-A- 7 303 692
- JP-A- 63 181 756
- JP-A- 2001 049 018
- JP-A- 2001 129 073
- JP-A- 2002 272 832
- VILJANEN V V ET AL: "Producing vascularized bone by heterotopic bone induction and guided tissue regeneration: a silicone membrane-isolated latissimus dorsi island flap in a rat model" JOURNAL OF RECONSTRUCTIVE MICROSURGERY, THIEME, NEW YORK, NY, US, vol. 13, no. 3, April 1997 (1997-04), pages 207-214, XP002974045 ISSN: 0743-684X
- MIZUMOTO S ET AL: "Fabrication of vascularized bone grafts using ceramic chambers" JOURNAL OF RECONSTRUCTIVE MICROSURGERY 1993 UNITED STATES, vol. 9, no. 6, 1993, pages 441-450, XP009049378 ISSN: 0743-684X
- TERHEYDEN H ET AL: "Mandibular reconstruction with a prefabricated vascularized bone graft using recombinant human osteogenic protein-1: an experimental study in miniature pigs. Part I: Prefabrication." INTERNATIONAL JOURNAL OF ORAL AND MAXILLOFACIAL SURGERY. OCT 2001, vol. 30, no. 5, October 2001 (2001-10), pages 373-379, XP009049383 ISSN: 0901-5027
- DUNNEN DEN W F A ET AL: "LONG-TERM EVALUATION OF NERVE REGENERATION IN A BIODEGRADABLE NERVE GUIDE" MICROSURGERY, NEW YORK, NY, US, vol. 14, 1993, pages 508-515, XP009017760 ISSN: 0738-1085
- DATABASE COMPENDEX [Online] ENGINEERING INFORMATION, INC., NEW YORK, NY, US; KIKUCHI M ET AL: "In vitro test and application for guided bone regeneration of beta tricalcium phosphate/poly-(lactide-glycolic acid-epsilon caprolactone) composites" XP002332976 Database accession no. E2001075469407 & KEY ENG MAT; KEY ENGINEERING MATERIALS 2001 TRANS TECH PUBL, UETIKON-ZUERICH, SWITZERLAND, vol. 192-195, 22 November 2000 (2000-11-22), pages 677-680,
- HOKUGO AKISHIGE ET AL: "Prefabrication of vascularized bone graft using guided bone regeneration" TISSUE ENGINEERING, vol. 10, no. 7-8, July 2004 (2004-07), pages 978-986, XP002332975 ISSN: 1076-3279

## Description

### Technical Field

The present invention relates to a graft material for bone regeneration and its production method that is used when regenerating a missing portion of bone .

### Background Art

In recent years, missing portions of body tissue have been able to be restored by regenerating body tissue as a result of filling a filling material into a missing portion of body tissue that has resulted due to tumor extraction or injury and so forth. Cells, growth factor, scaffold (support) and so forth are required for regeneration of body tissue, and a support in the form of a bone filling material is particularly indispensable for the regeneration of bone tissue. Although known examples of bone filling materials include hydroxyapatite (HAP) and tricalcium phosphate (TCP), in consideration of preventing foreign substances from remaining in the body, a scaffold material composed of a calcium phosphate porous body like β-TCP, for example, is used. When β-TCP is filled into a missing portion of bone, so-called remodeling takes place in which osteoclasts absorb the β-TCP and osteoblasts form new bone. Namely, the bone filling material that is filled into a missing portion of bone is replaced with the patient's own bone over time.

On the other hand, in order to accelerate the rate of restoration of a missing portion of bone following surgery, the use of cultured bone has been proposed which is produced by culturing bone marrow mesenchymal cells collected from the patient in vitro together with a bone filling material. Since cultured bone, which contains numerous bone marrow mesenchymal cells that have been grown by culturing using the bone filling material as a scaffold, is filled into a missing portion of bone, in comparison with methods in which only bone filling material is filled, the number of days until the cultured bone is replaced with the patient's own bone can be reduced considerably (see, for example, Document 1).

Document 1: Uemura, et al.: "Tissue Engineering in Bone Using a Biodegradable β-TCP Porous Material - Osferion: A New Material that Increase Strength in the Body", Medical Asahi, The Asahi Shimbun, October 1, 2001, Vol. 30, No. 10, p. 46-49.

In this case, it is also known that growth can be accelerated by supplying a growth factor such as cytokine.

Besides the use of artificial bone and cultured bone, another method for restoring a missing portion of bone is an autotissue graft such as grafting of the fibula with the vascular stalk intact. Since this grafting of the fibula with the vascular stalk intact uses a viable blood vessel, the quality of the take following transplant is known to be high as compared with ordinary autotissue grafts.

However, the phenomenon of ectopic ossification cannot be obtained in the case of grafting only artificial bone either subcutaneously or intramuscularly at a location other than bone.

In addition, in the case of grafting cultured bone, although the favorable phenomenon of ectopic ossification is observed, there are problems such as the actual taking of the grafted portion requiring considerable time due to such factors as requiring time to culture bone in vitro, problems relating to ensuring safety attributable to the culturing procedure, and the occurrence of vascularization following transplant.

Moreover, although grafting of the fibula with the vascular stalk intact results in satisfactory taking of the cultured bone, there are also various problems including the creation of a bone deficit at the portion where the bone was collected, limitations on the amount of bone collected, and a high degree of invasiveness for the patient.

In consideration of the circumstances described above, the object of the present invention is to provide a graft material for body tissue regeneration and its production method that enables adequate body tissue formation action to take place continuously in a body tissue filling material.

Other documents in this field include the following journal articles:
"Producing Vascularized Bone By Heterotopic Bone Induction And Guided Tissue Regeneration: A Silicone Membrane-Isolated Latissiumus Dorsi Island Flap In A Rat Model" by Velkko V. Viljanan, et al., Journal of Reconstructive Microsurgery, Thieme, New York, Vol. 13, No. 3, April 1997, pp 207-214;
"Fabrication Of Vascularized Bone Grafts Using Ceramic Chambers" by S. Mizumoto et al., Journal of Reconstructive Microsurgery, Vol. 9, No. 6, 1999, pp. 441-450; and
"Mandibular Reconstruction With A Prefabricated Vascularized Bone Graft Using Recombinant Human Osteogenic Protein-1: An Experimental Study In Miniature Pigs. Part I: Prefabrication" by Terheyden et al., International Journal of Oral and Maxillofacial Surgery, Vol. 30, No. 5, October 2001, pp. 373-379.

### Disclosure of the Invention

The present invention provides the following means to achieve the aforementioned object.

The present invention provides a graft material for bone regeneration as defined in claim 1. Preferred features of the invention are recited in the dependent claims. Thus, the graft material for bone regeneration comprises: a tubular member composed of a material that allows at least either oxygen or nutrients to permeate to the outside from a liquid that passes therein, a body tissue filling material arranged around the periphery of the tubular member, and a film composed of a biocompatible material that covers them.

According to this invention, when a liquid containing at least oxygen or nutrients is allowed to pass through the inside of the tubular member, at least the oxygen or nutrients contained in the liquid pass through the tubular member and is supplied to the body tissue filling material arranged around the periphery thereof. By mixing bone marrow or bone fragments inside the body tissue filling material or seeding the inside of the body tissue filling material with cells such as bone marrow cells, at least either oxygen or nutrients or both can continued to be supplied to the cells through the tubular member. In addition, by covering the body tissue filling material with a sheet composed of a biocompatible material, the desired body tissue can be efficiently regenerated in the body tissue filling material without the body material filling material having an effect on other body tissue arranged outside it even in a state when arranged in the body.

Furthermore, the cross-sectional shape of the tubular member is not limited to a circle, oval and so forth.

In addition, the present invention provides the graft material for body tissue regeneration according (1) above wherein, the body tissue filling material is composed of a granular ceramic porous body, and the film is wrapped around and sutured to the body tissue filling material while leaving both ends of the tubular member exposed.

According to this invention, at least oxygen or nutrients within liquid that has passed through the inside of the tubular member pass through the gaps in the granular ceramic porous body, permeate throughout the body tissue filling material, and are supplied to bone marrow or bone fragments mixed into the ceramic porous body or to cells that have been seeded therein. Since the sheet encloses the body tissue filling material in the state in which both ends of the tubular member are exposed, the granular body tissue filling material is held so as not to spill from the sheet. In addition, by connecting a liquid supply line and discharge line to both ends of the tubular member, liquid containing at least oxygen or nutrients can continuously be passed through the inside of the tubular member.

Thus, by covering the body tissue filling material with biocompatible sheet, a desired body tissue can be efficiently regenerated in the body tissue filling material surrounded by the sheet without having an effect on other body tissue arranged to the outside thereof even in a state in which it is arranged in the body.

According to this invention, at least oxygen or nutrients are supplied to a ceramic porous body from a blood flowing through a blood vessel or artificial blood vessel through that vessel and so forth. By mixing bone marrow or bone fragments inside the ceramic porous body or seeding the ceramic porous body with bone marrow cells and so forth, body tissue can be regenerated while continuing to supply at least oxygen or nutrients or both to the cells.

In addition, the present invention provides the graft material for body tissue regeneration according to (3) above wherein, bone marrow is mixed into the ceramic porous body.

According to this invention, mesenchymal stem cells contained in bone marrow cells can be grown with at least oxygen or nutrients or both supplied from a blood vessel and so forth, and body tissue can be regenerated by using the ceramic porous body as a scaffold.
(5) Moreover, the present invention provides the graft material for body tissue regeneration according to (3) or (4) above wherein, a growth factor is mixed into the ceramic porous body.

According to this invention, cell growth within the ceramic porous body is accelerated due to the action of the growth factor, and body tissue can be regenerated rapidly.

Moreover, the present invention provides an in vitro production method of a graft material for bone regeneration comprising:
arranging a bone filling material around the periphery of a tubular member composed of a material that allows at least either oxygen or nutrients to permeate to the outside from a liquid that passes therein, and covering the periphery of the body tissue filling material with a film composed of a biocompatible material.

According to this invention, since a graft material for body tissue regeneration can be produced merely by arranging a body tissue filling material around the periphery of a tubular member, and covering the periphery of the body tissue filling member with a film composed of a biocompatible material, a graft material for body tissue regeneration can be easily produced in the surgical setting, and be implanted directly in the body at the affected site or proximity thereto.

According to this invention, at least oxygen or nutrients is supplied to the ceramic porous body from blood flowing through the blood vessel or artificial blood vessel through the blood vessel and so forth. By then mixing bone marrow or bone fragments into the ceramic porous body or seeding the ceramic porous body with bone marrow cells and so forth, body tissue can be regenerated while continuing to supply at least oxygen or nutrients to the cells.

### Brief Description of the Drawings

Fig. 1 is a perspective view showing a graft material for body tissue regeneration as claimed in an embodiment of the present invention.
Fig. 2 is a longitudinal cross-sectional view showing the graft material for body tissue regeneration of Fig. 1.
Fig. 3A and 3B are photomicrographs depicting ectopic ossification six weeks after surgery using a graft material for body tissue regeneration as claimed in another embodiment of the present invention.
Fig. 4A and 4B are comparative examples of Fig. 3A and 3B in the form of a photomicrograph depicting ectopic ossification in the case of not using a bone filling material.
Fig. 5A and 5B are photomicrographs depicting ectopic ossification nine weeks after surgery using the graft material for body tissue regeneration of Fig. 3A and 3B.

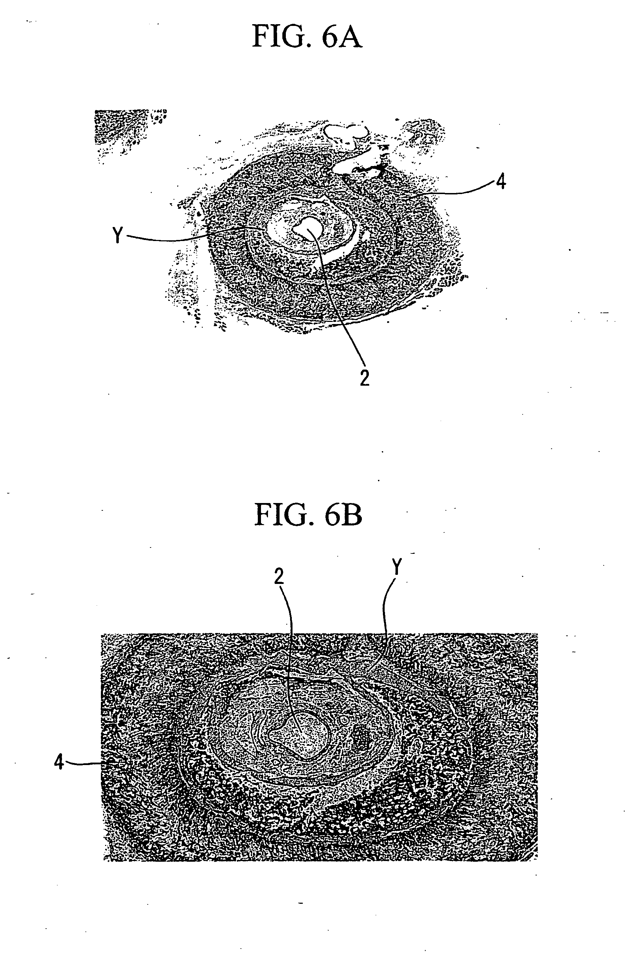

Fig. 6A and 6B are comparative examples of Fig. 5A and 5B in the form of a photomicrograph depicting ectopic ossification in the case of not using a bone filling material.
Fig. 7 is a graph showing the relationship between elapsed time after surgery and bone matrix density.
Fig. 8 is a photomicrograph depicting vascular growth within the graft material for body tissue regeneration of Fig. 3.

### Brief Explanation of the Reference Symbols

A Liquid, blood
B Nutrients, etc.
1,10 Graft material for bone regeneration (graft material for body tissue regeneration)
2 Tubular member, blood vessel, artificial blood vessel
3 Body tissue filling material (bone filling material)
4 Biocompatible film (film)

### Best Mode for Carrying Out the Invention

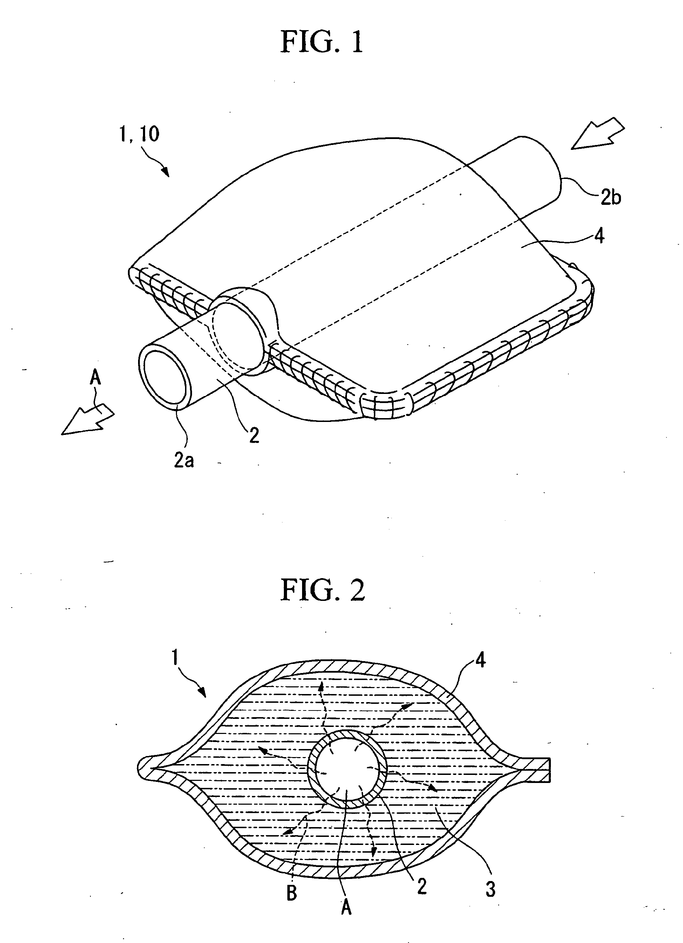

The following provides an explanation of a graft material for body tissue regeneration as claimed in an embodiment of the present invention with reference to Fig. 1.

The graft material for body tissue regeneration as claimed in the present embodiment is a graft material for bone regeneration 1 for regenerating body tissue in the form of bone, and as shown in Figs. 1 and 2, is composed of a tubular member 2, a bone filling material 3 arranged so as to surround the periphery of the tubular member 2, and a film 4 composed of a superior biocompatible material (to be referred to as a biocompatible film) that encloses the bone filling material 3.

Furthermore, there are no particular limitations on the superior biocompatible material, and materials that are absorbed by the body or those that are not absorbed by the body may be used. Examples of materials that are absorbed by the body include polylactic acid, polyglycolic acid, polylactone, polycaprolactone or their copolymers, collagen, chitin and chitosan. Materials that are absorbed by the body offer the advantage of not leaving behind any foreign substances around the regenerated bone since they are absorbed into the body over time. On the other hand, although materials represented by PTFE that are not absorbed by the body remain in the form of foreign substances for a long period of time, they are able to provide a satisfactory environment for bone formation.

The aforementioned tubular member 2 is composed of a material that allows at least oxygen and nutrients (to be referred to as nutrients B) to be supplied to the outside by permeating through tubular member 2 from a liquid A such as blood that flows therein in the manner of, for example, a hollow membrane composed of an osmotic membrane or reverse osmotic membrane.

The aforementioned bone filling material 3 is composed of, for example, granules of a β-TCP porous body.

Biocompatible film 4 is arranged with tubular member 2 in the center and as a result of enclosing bone filling material 3 with bone filling material 3 arranged around the periphery of tubular member 2 and suturing the outside, is formed into the shape of a tube or pouch. As a result, a graft material for bone regeneration 1 is composed that prevents leakage of bone filling material 3, is covered with a superior biocompatible film 4 for providing an environment (site) that allows regeneration of a desired body tissue without being affected by the surrounding environment, and only both ends 2a and 2b of tubular member 2 are exposed to the outside from biocompatible membrane 4.

The following provides an explanation of the action of a graft material for bone regeneration 1 as claimed in the present embodiment composed in this manner.

In order to regenerate a missing portion of bone with the graft material for bone regeneration 1 as claimed in the present embodiment, a portion of a blood vessel exposed by an incision is severed, and the ends of said blood vessel are sutured to both ends 2a and 2b of the aforementioned tubular member 2. As a result, blood A is allowed to flow through the inside of tubular member 2. In addition, bone marrow and bone fragments or bone marrow cells are mixed within bone filling material 3 of bone regeneration graft material 1 by penetrating biocompatible film 4 using, for example, a syringe. Furthermore, a blood vessel of the body (e.g., arteriovenous fascicle) or muscle fascicle can be used instead of the aforementioned tubular member 2. For example, in the case of using an arteriovenous fascicle instead of tubular member 2, bone filling material 3 is arranged around the periphery of the exposed arteriovenous fascicle and biocompatible film 4 is arranged as to encase bone filling material 3 followed by suturing. A muscle fascicle can be used in the same manner as this arteriovenous fascicle.

While in this state, the bone regeneration graft material 1 is arranged intramuscularly or subcutaneously followed by suturing the skin incision and allowing to stand for several weeks.

Since the mixture of bone marrow and bone fragments or bone marrow cells injected into bone filling material 3 is maintained in a state in which it is isolated from outside muscle tissue or subcutaneous tissue by biocompatible film 4, it is not affected by these tissues, and ectopic ossification action occurs by using bone filling material 3 as a scaffold within the muscle tissue or subcutaneous tissue.

In this case, since tubular member 2 is composed of a material that allows nutrients B within blood A passing through tubular member 2 to permeate tubular member 2, cells contained in bone marrow that generate bone formation action within bone filling material 3 grow rapidly and reliably by obtaining nutrients B continuously supplied from blood A, and are composed in the form of a healthy regenerative bone graft.

The regenerative bone graft composed in this manner is excised by making another incision into the skin and then filled into a missing portion of bone. In the case biocompatible film 4 is made of a material that is absorbed by the body, it is absorbed by the body and disappears over time. Thus, since the regenerative bone graft is no longer covered by biocompatible film 4, bone formation action also occurs on the outer surface due to the action of bone formation factors present in the bone tissue outside, thereby resulting in reconstruction of the missing portion by adapting to the missing portion of bone.

At this time, by maintaining the blood vessel connected to tubular member 2 in the connected state during the stage of subcutaneous growth, bone formation action can be accelerated and the missing portion of bone can be filled in rapidly and adequately as a result of continuously supplying nutrients B from blood A even during bone formation within the missing portion of bone.

Furthermore, in the case of using a blood vessel (e.g., arteriovenous fascicle) or muscle fascicle of the body instead of the aforementioned tubular member 2, and particularly in the case of using an arteriovenous fascicle, a new vasoganglion is formed that is continuous with the arranged vessel 2 in the bone tissue regenerated within the graft material for body tissue regeneration. This newly formed vasoganglion is provided with a blood flow, and a bone graft can be provided that has newly formed blood vessels at the time of grafting. In order to realize early bone adhesion following grafting, this newly formed vasoganglion is extremely important, and enables reconstruction in the same manner as an autobone graft with a newly formed vascular stalk intact.

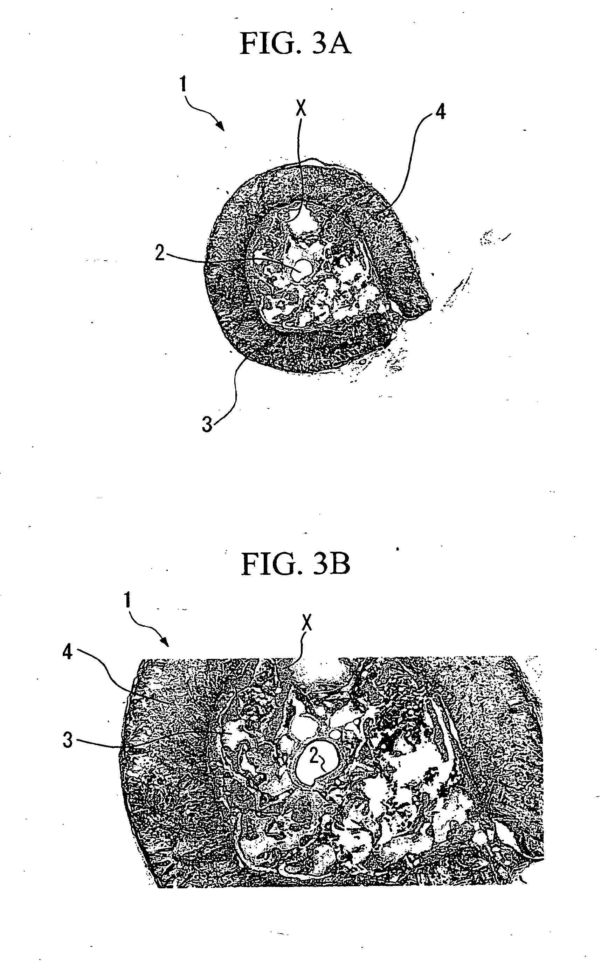

Next, an explanation is provided of a graft material for body tissue regeneration as claimed in another embodiment of the present invention with reference to Figs. 3(A, B) through 8.

Graft material for bone regeneration 10 as claimed in the present embodiment differs from graft material for bone regeneration 1 as claimed in the aforementioned embodiment in that a blood vessel (arteriovenous fascicle) is used for tubular member 2, and a PLC (polylactide-ε-caprolactone) film is used for biocompatible film 4.

In order to compose graft material for bone regeneration 10 as claimed in the present embodiment, an incision is made in the skin, blood vessel 2 is withdrawn from subcutaneous tissue or muscle tissue, blood vessel 2 is enclosed in biocompatible film 4 in the state in which a granular bone filling material 3 is arranged around the periphery of blood vessel 2, and biocompatible membrane 4 is sutured to as to isolate bone filling material 3 from the outside. As a result, since graft material for bone regeneration 10 is composed in the same manner as the aforementioned embodiment, it can be implanted in the body in the state in which bone marrow, bone marrow cells or a mixture thereof is injected into bone filling material 3 with a syringe and so forth.

According to this graft material for bone regeneration 10 as claimed in the present embodiment composed in this manner, since the need to suture blood vessel can be eliminated, the grafting procedure can be made easier. Rapid and adequate ectopic ossification action can then be promoted by using bone filling material 3 as a scaffold in the state in which nutrients B are supplied through blood vessel 2 to cells contained in the bone marrow within bone filling material 3 from blood A flowing therein while being isolated from outside tissue by biocompatible film 4.

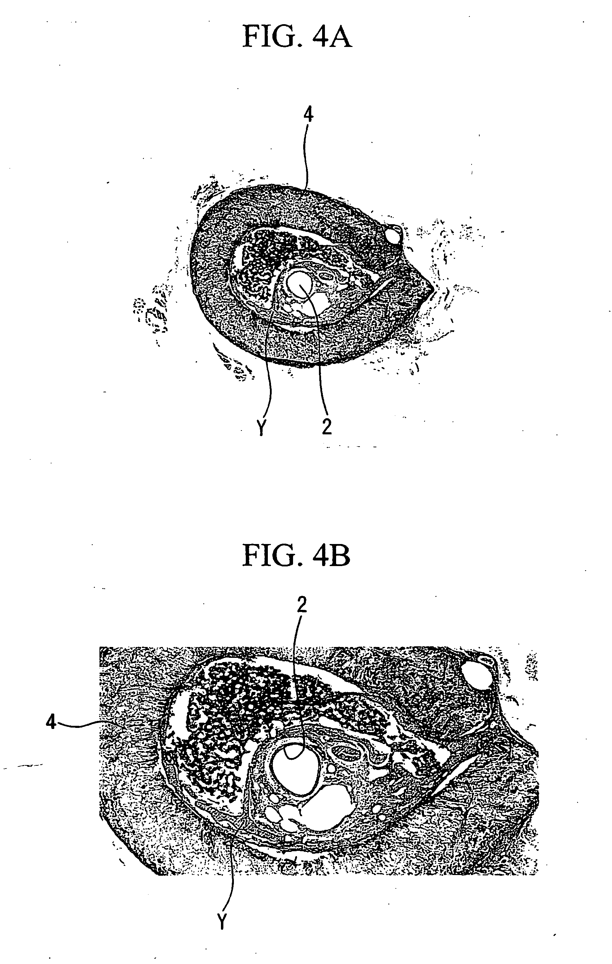

Fig. 3A is a drawing showing a photomicrograph of a longitudinal cross-section depicting ectopic ossification of bone regeneration graft material 10 as claimed in the present embodiment in which bone filling material 3 in the form of β-TCP granules are surrounded by biocompatible film 4 in the state in which six weeks have elapsed following surgery in which the graft material was placed in muscle tissue. Fig. 3B shows an enlarged view of this. Fig. 4A and 4B are photomicrographs similar to Fig. 3A and 3B that depict ectopic ossification of a graft material for body tissue regeneration in the case of not using bone filling material 3.

In looking at these figures, in the case of bone regeneration graft material 10 as claimed in the present embodiment, as indicated by symbol X, ectopic ossification effects can be seen to be occurring uniformly not only around the periphery of blood vessel 2 appearing in white in the center, but also throughout the entire space within biocompatible film 4, and bone tissue can be seen to be forming. In contrast, in the case of not using bone filling material 3, as indicated by symbol Y, bone formation effects can be seen to only be occurring on the surface of biocompatible film 4 and on the surface of the filled substance, while healthy ectopic ossification can be seen to not be taking place.

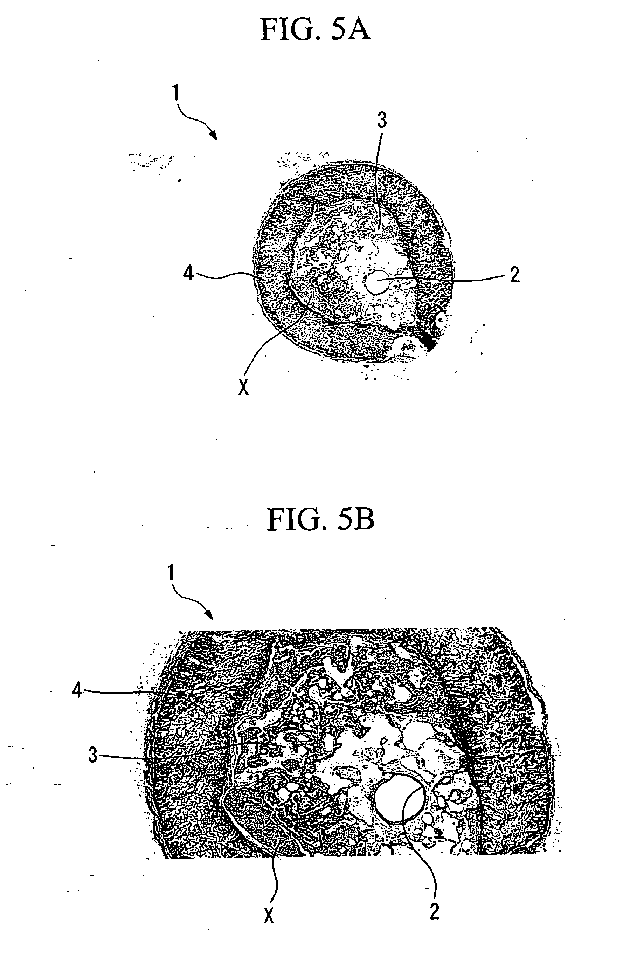

Figs. 5(A, B) and 6(A, B) are similar photomicrographs depicting the status at nine weeks after surgery. According to these photomicrographs, in the case of bone regeneration graft material 10 as claimed in the present embodiment, bone tissue X can be seen to be forming over a wide range in the space within biocompatible film 4.

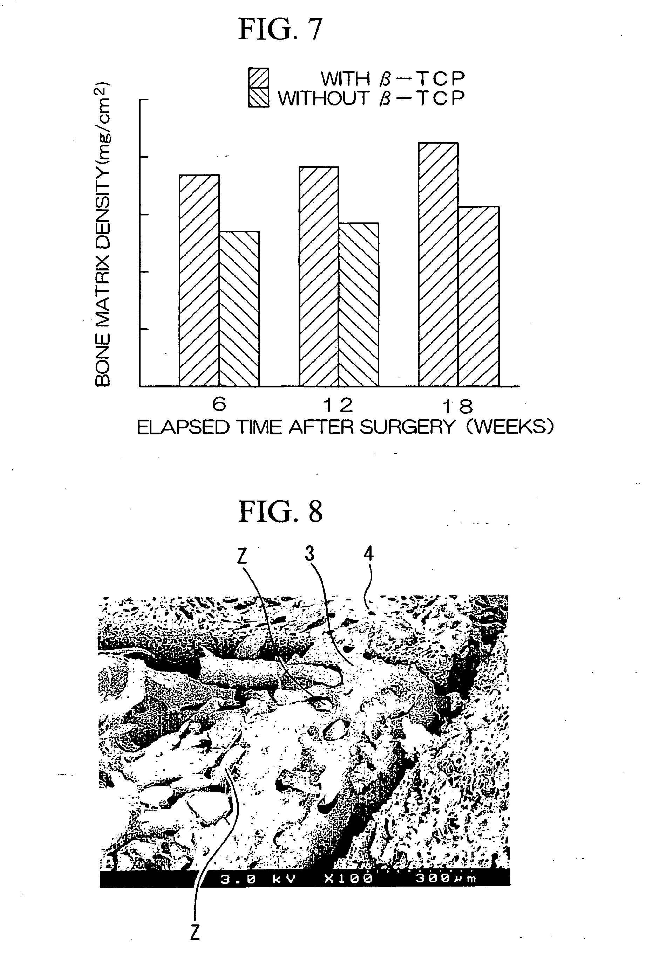

Fig. 7 is a graph showing the relationship between elapsed time (weeks) after surgery and bone matrix density with respect to the state of ectopic ossification. According to this graph, the bone formation action of cells that have used bone filling material 3 as a scaffold is remarkable.

A regenerative bone graft composed in this manner is excised from subcutaneous tissue or muscle tissue in a state in which the blood vessel or tubular member 2, which allows nutrients and so forth to reach the bone tissue, is still provided, thereby making it possible to graft the regenerative bone graft into a missing portion of bone in the form of viable bone that retains blood flow.

As a result, the effect is achieved in which the regenerative bone graft is able to satisfactorily take to the surrounding bone tissue without being affected by the blood flow of the surrounding bone tissue at the missing portion of bone where the regenerative bone graft is to be placed. In addition, capillaries are able to develop from blood vessel 2 arranged in the center of bone filling material 3 across the gaps in bone filling material 3, thereby allowing nutrients and so forth to be distributed throughout the space within biocompatible film 4. Fig. 8 is a photomicrograph of a longitudinal cross-section of a bone graft showing a capillary Z that has developed in a gap of bone filling material 3.

In this manner, according to bone regeneration graft material 10 as claimed in the present embodiment, since the bone graft itself has its own blood flow, it is resistant to infection and it can be grafted into a missing portion of bone having poor circulation. In addition, this bone regeneration graft material 10 can be applied to a comparatively wide range of missing portions of bone depending on the grafted bone, and due to its rapid adhesion to bone, rehabilitation can progress to the state at which loading is started more quickly.

Moreover, according to bone regeneration graft material 10 as claimed in the present embodiment, there is also the effect of being able to minimize the invasiveness to the patient while obtaining similar effects as bone grafting with the vascular stalk intact as previously mentioned. Namely, since viable bone tissue retaining blood flow is sampled from another healthy site and graft to a missing portion of bone in the case of ordinary bone grafting with the vascular stalk intact, invasion of a healthy site cannot be avoided. However, according to the bone regeneration graft material 10 as claimed in the present embodiment, since a bone graft in which ectopic ossification has been induced at a site other than a missing portion of bone can be filled into a missing portion of bone, invasion of a healthy site can be prevented. Thus, together with being able to shorten the duration of the surgical procedure, restrictions on its application such as general condition of the patient and so forth can be eliminated.

Furthermore, although an explanation of the aforementioned embodiment used the example of bone tissue for the body tissue in the case of using a mixture of bone marrow and bone fragments or bone marrow cells, the mixture may be extracted not only from bone marrow, but also peripheral blood or placental blood. In addition, the mixture is not limited to a mixture of bone marrow and bone fragments or bone marrow cells, but rather somatic cells such as ES cells, somatic stem cells, osteocytes, chondrocytes or nerve cells may also be used. In addition, the present invention may be used to regenerate bone tissue, but also any body tissue such as cartilaginous tissue, muscle tissue or subcutaneous tissue.

In addition, a growth factor such as cytokine, concentrated platelets, BMP, FGF, TGF-β, IGF, PDGF, VEGF, HGF, their compounds or other substances contributing to growth may be supplied to promote the growth of cells supplied to the body tissue filling material. In addition, estrogen and other hormone preparations as well as vitamins and other nutrients may also be supplied.

At this time, the genes of these physiologically active substances or gradual release materials of physiologically active substances and so forth may also be used.

In addition, although the explanation of the aforementioned embodiments used the example of a biocompatible film for the film having biocompatibility, this film is not limited to this, but rather a film may be used that is composed of any arbitrary biocompatible material such as a collagen film.

### Industrial Applicability

As has been explained above, according to the graft material for body tissue regeneration and its production method as claimed in this invention, by allowing nutrients from a liquid such as blood that passes through the inside of a tubular member to permeate to the outside, cell growth can be promoted by allowing the cells to use a bone filling material as a scaffold, thereby demonstrating the effect of being able to rapidly produce a viable graft material for body tissue regeneration having its own blood flow.

In addition, according to a graft material for body tissue regeneration produced in this manner, in addition to being resistant to infection and being able to be grafted in a comparatively wide range of missing portions of bone, the graft material is able to satisfactorily take to body tissue surrounding a missing portion of bone regardless of the status of that surrounding body tissue, thereby demonstrating the effect of being able to minimize invasiveness to the patient while also shortening the duration of surgery.

## Claims

1. A graft material (1) for bone regeneration, comprising:
a tubular member (2) for conveying a liquid containing at least oxygen or nutrients, the tubular member (2) composed of a material that allows at least oxygen or nutrients to permeate to the outside from the liquid that passes therein,
a bone filling material (3) arranged around the periphery of the tubular member (2),
**characterized by** a film (4) composed of a biocompatible material that covers the tubular member (2) and the bone filling material (3) in the form of a granular ceramic porous body, wherein the film (4) is wrapped around and sutured to the bone filling material and encloses the bone filling material (3) and both ends (2a, 2b) of the tubular member (2) are exposed.

2. A graft material (1) for bone regeneration according to claim 1, wherein bone marrow and bone fragments are mixed into the ceramic porous body.

3. A graft material (1) for bone regeneration according to claim 1 or 2, wherein a growth factor is mixed into the ceramic porous body.

4. An in vitro production method of a graft material for bone regeneration comprising:
arranging a bone filling material (3) around the periphery of a tubular member (2) composed of a material that allows at least oxygen or nutrients to permeate to the outside from a liquid that passes therein, and
covering the periphery of the bone filling material (3) with a film (4) composed of a biocompatible material,
**characterized in that** the film (4) is wrapped around and sutured to the bone filling material and encloses the bone filling material (3) in the form of a granular ceramic porous body while leaving both ends (2a, 2b) of the tubular member (2) exposed.

## Patentansprüche

1. Implantationsmaterial (1) zur Regenerierung von Knochen, aufweisend:
ein rohrförmiges Element (2) zum Transport einer Flüssigkeit, die mindestens Sauerstoff oder Nährstoffe enthält, wobei das rohrförmige Element (2) aus einem Material besteht, das für mindestens Sauerstoff oder Nährstoffe durchlässig ist, so dass diese aus der durchfließenden Flüssigkeit nach außen gelangen können,
ein Knochenfüllmaterial (3), das um den Umfang des rohrförmigen Elements (2) angeordnet ist,
**gekennzeichnet durch** einen Film (4), der aus einem biokompatiblen Material besteht, das das rohrförmige Element (2) und das Knochenfüllmaterial (3) in Form eines granularen porösen Keramikkörper bedeckt, wobei der Film (4) um das Knochenfüllmaterial (3) gewickelt, mit dem Knochenfüllmaterial vernäht ist und das Knochenfüllmaterial (3) einschließt und die beiden Enden (2a, 2b) des rohrförmigen Elements (2) freiliegen.

2. Implantationsmaterial (1) zur Regenerierung von Knochen nach Anspruch 1, bei dem Knochenmark und Knochenfragmente in den porösen Keramikkörper gemischt sind.

3. Implantationsmaterial (1) zur Regenerierung von Knochen nach Anspruch 1 oder 2, bei dem ein Wachstumsfaktor in den porösen Keramikkörper gemischt ist.

4. *In vitro*-Herstellungsverfahren für ein Implantationsmaterial zur Regenerierung von Knochen, aufweisend:
Anordnen eines Knochenfüllmaterials (3) um den Umfang des rohrförmigen Elements (2), das aus einem Material besteht, das für mindestens Sauerstoff oder Nährstoffe durchlässig ist, so dass diese aus der durchfließenden Flüssigkeit nach außen gelangen können, und
Abdecken des Umfangs des Knochenfüllmaterials (3) mit einem Film (4), der aus einem biokompatiblen Material besteht,
**dadurch gekennzeichnet, dass** der Film (4) um das Knochenfüllmaterial (3) gewickelt und mit diesem vernäht ist und das Knochenfüllmaterial (3) in Form eines granularen porösen Keramikkörper bedeckt, während die beiden Enden (2a, 2b) des rohrförmigen Elements (2) freiliegend bleiben.

## Revendications

1. Matériau de greffe (1) pour une régénération osseuse, comprenant :
un élément tubulaire (2) pour transporter un liquide contenant au moins de l'oxygène ou des nutriments, l'élément tubulaire (2) composé d'un matériau qui permet qu'au moins l'oxygène ou les nutriments s'infiltrent vers l'extérieur à partir du liquide qui passe dans celui-ci,
un matériau d'obturation osseuse (3) agencé autour de la périphérie de l'élément tubulaire (2),
**caractérisé par** un film (4) composé d'un matériau biocompatible qui recouvre l'élément tubulaire (2) et le matériau d'obturation osseuse (3) sous la forme d'un corps poreux céramique granulaire, dans lequel le film (4) est enveloppé autour du matériau d'obturation osseuse et suturé sur celui-ci et enferme le matériau d'obturation osseuse (3) et les deux extrémités (2a, 2b) de l'élément tubulaire (2) sont exposées.

2. Matériau de greffe (1) pour une régénération osseuse selon la revendication 1, dans lequel de la moelle osseuse et des fragments d'os sont mélangés à l'intérieur du corps poreux céramique.

3. Matériau de greffe (1) pour une régénération osseuse selon la revendication 1 ou 2, dans lequel un facteur de croissance est mélangé à l'intérieur du corps poreux céramique.

4. Procédé de production in vitro d'un matériau de greffe pour une régénération osseuse comprenant :
l'agencement d'un matériau d'obturation osseuse (3) autour de la périphérie d'un élément tubulaire (2) composé d'un matériau qui permet qu'au moins de l'oxygène ou des nutriments s'infiltrent vers l'extérieur à partir d'un liquide qui passe dans celui-ci, et
le recouvrement de la périphérie du matériau d'obturation osseuse (3) avec un film (4) composé d'un matériau biocompatible,
**caractérisé en ce que** le film (4) est enveloppé autour du matériau d'obturation osseuse et suturé sur celui-ci et enferme le matériau d'obturation osseuse (3) sous la forme d'un corps poreux céramique granulaire tout en laissant les deux extrémités (2a, 2b) de l'élément tubulaire (2) exposées.
